# EUROPEAN PATENT SPECIFICATION

(11) **EP 2 387 320 B1**
(45) Date of publication and mention of the grant of the patent: **06.11.2013**
(21) Application number: 10700595.1
(22) Date of filing: 14.01.2010
(51) Int. Cl.: A21D 13/00, A23L 1/00, A23L 1/164, A23L 1/305, A23L 1/308, A21D 2/18, A21D 2/26, A21D 2/36, A23P 1/14, A21D 6/00, A21D 8/02

(54) **EXPANDED FOOD PRODUCT AND PROCESS OF PREPARATION**
AUFGEBLÄHTES LEBENSMITTELPRODUKT UND HERSTELLUNGSVERFAHREN
PRODUIT ALIMENTAIRE EXPANSE ET PROCEDE DE PREPARATION

(30) Priority: 14.01.2009 GB 0900551
(43) Date of publication of application: 23.11.2011
(73) Proprietor: Carritech Research Limited, Belfast BT1 5HB (GB)
(72) Inventor: Horton, Richard, Belfast, BT9 6LG (GB)
(74) Representative: Williams, Richard Andrew Norman
(86) International application number: PCT/GB2010/050048
(87) International publication number: WO 2010/082053

(56) References cited:
- EP-A2- 0 316 145
- WO-A1-86/06256
- WO-A1-99/22607
- WO-A1-2009/076131
- WO-A2-00/78163
- WO-A2-02/062153
- GB-A- 1 495 194
- US-A- 3 650 769
- US-A- 5 523 106
- US-A1- 2006 019 009

## Description

The present invention relates to processes for producing compositions that are ingestible by humans or animals and may be consumed by them. The compositions can include nutrients and/or pharmaceuticals. The compositions can be used as food, food ingredients, health, dietary, or nutrition supplements and/or as pharmaceuticals. The processes for producing the compositions involve combining chosen ingredients to produce compositions which can be expanded and/or dried under vacuum at controlled temperatures to produce food products with an internal structure of air pockets.

A problem with food products that have undergone a processing procedure is that certain ingredients can be subjected to heat and other processing conditions which reduce or destroy their nutritional value to the consumer. There is an increasing realisation of this as public awareness of the need for balanced and healthy diet grows. In this vein, there is also an escalating trend to try to reduce the amount of processed foods in the daily diet. Yet despite this, people still desire to have the convenience purchase and pleasure of eating prepared and factory produced foods and snacks.

Cooking or baking mixtures of ingredients to make a processed food product can result in undesirable flavours being imparted to the resulting food product. One example is fish omega-3 oil which produces a fishy odour when subjected to heating. This can taint processed food products including this ingredient.

In the manufacture of confectionery products, "chocolate crumb" is an intermediate used to make chocolate. A well known process for preparing chocolate crumb combines mixing sugar, milk powder, cocoa mass, water and other optional additives at around 50°C to 70°C. This forms a crumb paste containing between 5% and 10% water. This paste is viscous and sticky and is formed into a thin slab on a moving belt and passed into an oven. Here the slab is heated under vacuum for between 30 and 120 minutes at a temperature between 50°C and 130°C until the water content reduces to about 2% or less. The dried slab is broken into small pieces which may advantageously be stored or transported, or used immediately to make chocolate.

EP 1245158 describes a process for the tailoring of flavour in a chocolate crumb. Temperature, time and water content are adjusted as desired. Milk solids, sugar, cocoa liquor and 1.2% to 8% total water content are mixed at a temperature of between 85 °C and 180 °C for 2.5 to 25 minutes, after which the mixture is dried to a moisture content of less than 3%. The mixing and heating of the composition can alternately be carried out in either the absence or presence of cocoa solids. Drying may be carried out in a vacuum for a period of 30 to 60 minutes. Drying temperatures range from 60 °C to 180°C.

WO2007/12529 describes another process for making chocolate crumb. A wet crumb feedstock comprised of bulk sweetener, protein and water is formed and simultaneously dried and comminuted by the action of a thin layer rotary paddle drier. The wet crumb is comprised of sugar, milk or milk powder/whey, cocoa mass and water. The granules are maintained at temperatures of between 10°C and 80°C to prevent them from adhering to each other. They are dried at atmospheric conditions not necessitating a vacuum due to their free-flowing behaviour and robustness. The invention described seeks to overcome undesirably long drying times for crumb when using vacuum drying, whether in a band dryer or box oven.

US2008/0020098 describes a process for producing a protein nutrition bar. A composition of greater than 50% of a non-soy protein, e.g. milk, rice or pea protein, preferably whey protein, is extruded into nuggets at a temperature in the range 60 °C to 140 °C. The temperature is selected to avoid damage to selected non-soy proteins and any concomitant off-taste. The extruded protein bars are then dried at atmospheric pressure in a belt/conveyor drier and/or a fluid bed drier. In a second described process, injection of supercritical CO₂ into the extruder is used as a way of lowering the extrusion temperature and at the same time forming a puffed product. The extrusion temperatures are in the range 75 °C - 90 °C, not more than 95°C.

US6607774 describes expanded edible products having a porous, foamed or cellular interior and a self sealing surface or skin. The ingredients used comprise a structure-enhancing hydrocolloid, water and a bulking agent. The hydrocolloid is described as critical to the invention and is a substance that undergoes gelation on adsorbing water.

The hydrocolloids include amylose or amylopectin, starches, gelatins, dextrins, pectins, gum arabic, alginates, carageenan gum, agar, locust bean gum, guar gum, xanthan gum, gellan gum and mixtures of these. At least one component of the pre-expanded formulation must be crystallisable, e.g. sugars, sugar alcohols, starch or cellulose. The crystallisable portion is cooked (i.e. 140 °C - 142 °C ) to form syrup and mixed with the hydrocolloid and any other additives to make a pre-expanded formulation. The formulation is placed in a vacuum expansion oven at temperatures in the range 80 °C - 83 °C.

US5523106 describes a shelf-stable, crispy snack made from fruit or vegetable juice or concentrate. Juice or concentrate is mixed with hydrolyzed starches, for example maltodextrin and gelatinised wheat starch. A dough is formed by kneeding, formed into shapes which are then subjected to vacuum drying to expand the composition and dry it to a shelf stable moisture content, without damaging the nutritive value, colour, odour and taste of the juice. The starch hydro lyzate and gelatinized starch create a glassy matrix together with the juice solids and impart a crispy texture to the final product.

DE10033733 A1 describes making a food paste from three or more of almost any food or food ingredient, including items such as bakery products, vegetables, meat, fish, cereals, seeds, egg, milk and so forth. The paste is divided up into suitable sized portions which have a core temperature not exceeding 50°C. The portions are then baked in a vacuum oven so they reach a core temperature in the range 18 °C - 90 °C. The baking process results in an expansion due to the evaporation of steam. Examples of the products produced are a vegetable bar made from vegetables, a fruit bar made from pureed fruit and sugar or a meat-flavoured snack made from meat and pieces of vegetable. What is disclosed is simply the homogenisation and heating of any foodstuff or combination of foodstuff by vacuum baking at lower temperatures (i.e. 18 °C - 90 °C) without regard to the resulting texture or structure of the product.

US3650769 describes a method of making friable, porous and instantly soluble food products using a saccharide dissolved in water as a matrix material. Finely divided edible food substances are added as filler to the matrix. The filler adsorbs water from the matrix and forms stiff syrup having the consistency of taffy. The filler material can be, for example, flour, dried milk, cocoa, coffee or powdered vegetable solids - all of which remain largely undissolved in the matrix. Saccharides such as sugar which result in a crystalline structure are not used. The taffy is formed into sheets and divided up into pieces of the desired size. These pieces are heated to soften them before being subjected to vacuum until expanded to two times their original size.

FR2750015 A1 describes a process for making a cheese product. A cheese-based starting material with a water content of 25 - 65% by weight is dried and expanded in a microwave oven under vacuum at a temperature of less than 40°C. Grains of cheese expanded in this way are turned into expanded cheese "popcorn". Bars of similar expanded cheese product are described.

JP60160845 A describes an expanded food product with a uniform and porous texture and having a thin outer wall. The pre-expanded mixture is made by adding starch to ethanol and then water. The mixture is heated and extruded under pressure. An expanded starch-type food product is made in which the ethanol causes expansion and acts as a preservative.

WO02/00201 describes a method of making pharmaceutical dosage forms which may take place *in situ* in a blister pack. They are made from an homogeneous solution or dispersion of pharmaceutically active agent in an excipient, such as maltodextrin. The dispersion is dried under vacuum at a low temperature to expand it, but without boiling taking place. The dosage forms are expanded, lamellar, porous, sponge or foam-like structures.

WO97/34503 describes a process for making expanded confectionery shapes. The main ingredients are malted milk and malt extract. In one process, bicarbonate is used as the expanding agent. The composition is extruded using an extrusion cooker at a temperature in the range 40 - 70 °C. In another process, gaseous or supercritical carbon dioxide or nitrogen or compressed air is injected into the pre-extrusion zone. Both bicarbonate and gas injection may be used together to expand the products into an ambient pressure environment.

WO02/37979 describes a process and apparatus for making expanded confectionery of three kinds. A first uses milk powder as the structuring material and includes glucose or malt extract plus bicarbonate and water. A second "sugar confectionery" contains predominantly sugars or sugar alcohols, plus a structuring material, e.g. starch, maltodextrin, gum, milk powder or gelatine, plus water, flavourings and colour. A third "chocolate confection" uses milk or milk product as the structuring material ingredient, plus sugar, water, cocoa and fat. The expandable formulations are formed into pieces by extrusion and then dried in a vacuum oven at temperatures which avoid denaturation of ingredients but at the same time expand the formulation evenly to give a rounded or spherical product.

GB 1495194 describes a process of making textured vegetable protein products from potato pulp. The ingredients used are potato pulp and water giving at least 20% protein and less than 2% fat on a dry weight basis. The process requires mixing the potato pulp and water at an elevated pressure and a temperature above 100 ºC to make a flowable substance. The hot mixture under pressure is then extruded through a restricted orifice into an environment of lower pressure and lower temperature which results in expansion. A textured product is obtained after allowing the expanded mixture to dry.

WO99/22607 describes a "ready to eat" cereal or snack product. At least partially ungelatinized grain is placed in a cooker extruder. The grain is worked by the screw of the cooker to mix, heat and liquefy the grain to form a plasticised mass. The mass is extruded through a die orifice, and at the point just prior to extrusion, temperatures are in the range 120 ºC - 280 ºC.

US2006/0019009 A1 describes a method of making a low carbohydrate puffed snack from ground corn, soy isolate and soy concentrate. The mixture of ingredients are placed in an extruder and forced through an exit die at a pressure of between about 600 -1400 psi (about 4137 kPa - about 9653 kPa) and a temperature between about 350 ºF to 425 ºF (about 177 ºC - about 218 ºC). The extrusion results in puffing and after drying there is a ready to eat low carbohydrate snack food.

W02009/076131 describes a method of making an extruded, textured protein material (e.g. in the form of "nuggets") for use in food products such as snack foods, snack puffs or breakfast cereals. The material contains a high concentration of vegetable protein and omega-3 fatty acids. The extrudable mixture is made from at least 50 wt% vegetable protein and about 5 wt% to about 15 wt% oil, both on a moisture free basis. At least 10 wt% of the oil is an omega-3 fatty acid. The mixture is pressurised to about 400 psi (about 2758 kPa) and then extruded at a temperature of at least 35 ºC.

An object of the present invention is to provide pleasant-to-eat delivery mechanisms for nutrients and/or medicines, particularly those nutrients and/or medicines that are damaged or destroyed by heat, so as to produce alternative 'health and weitness' foods.

Another object of the invention is to achieve the desired flavours of final products early in the production process at the ingredient mixing stage. The object is therefore to inhibit flavour development generated by temperatures used in conventional cooking processes (i.e. avoiding caramelization or browning of the ingredients).

A further object of the invention in the case of some recipes or formulations, is to avoid the formation of acrylamides generated via conventional cooking processes. Acrylamides are recognised to be harmful to human and animal health.

Another object of the invention is to reduce the carbon footprint of processed foods and snack foods and achieve energy and cost savings by using low temperature processing.

The inventor has discovered that particular ingredient formulations coupled with certain processes provide a substantially dried, shaped food product that has a porous structure of air pockets, which in certain embodiments are characterised as "honeycombed," and which preferably have a crunchy feel and sensation when consumed. The inventor has also discovered how to incorporate ingredients or substances into the expanded, porous food products in a way which preserves their natural biological activity or nutritional value or effects.

Part of the inventor's discovery was an unexpected finding that dietary fibre and/or hydrolysed wheat flour can be used as the matrix forming ingredient in a dough or paste formulation that when subjected to low temperature vacuum baking, generates a food product which has an internal structure of air pockets, including a "honeycomb" structure.

Accordingly, the present invention provides a method of producing an expanded food product having an internal structure of air pockets comprising:
(a) combining together ingredients comprising a matrix-forming ingredient selected from dietary fibre and/or hydrolysed wheat flour;
(b) adding one or more liquids to the ingredients to produce a dough, paste or moist powder at temperature not exceeding 75 °C;
(c) forming the dough or paste or moist powder into individual pieces;
(d) treating the pieces so that they attain a temperature not exceeding 75 °C, at which temperature evaporation of liquid and/or transition from solid to gas is sufficient to expand the pieces when subjected to at least a partial vacuum; and
(e) exposing the pieces to at least a partial vacuum and at a temperature not exceeding 75 °C such that liquid evaporates causing the matrix to expand to form the food product having an internal structure of air pockets.

The dietary fibre is preferably selected from one or more of soluble dietary fibre, prebiotic dietary fibre, polydextrose and soluble corn fibre,

The particular matrix-forming ingredients and their combinations are able to produce doughs or pastes which on vacuum expression may advantageously form a brittle structure of air pockets, e.g. a honeycomb structure.

The matrix-forming ingredient may further comprise whey protein isolate and/or whey protein concentrate. This ingredient advantageously does not interfere with the generation of the internal structure of air pockets, but allows a food product with a higher protein content to be made.

The ingredients may further comprise a non-structural ingredient, preferably a temperature-sensitive, non-structural ingredient. These ingredients may have no calorific and/or nutritive value. Such ingredients are preferably combined with the other ingredients to form the dough or paste. Non-structured ingredients can additionally or alternatively be added to the food product after expansion by a coating process, e.g. spraying,

The one or more matrix-forming ingredients are preferably present in the range 30% - 100 % by weight of total dry ingredients (i.e. excluding the one or more liquid ); more preferably 40% - 100% by weight of the said total dry ingredients. The matrix-forming ingredients may be present in a range (by percent of total dry ingredients) wherein one of the following lower range limits is combined with one of the following upper range limits.

Lower limits: 30%, 31%, 32%, 33%, 34%, 35%, 36%, 37%, 38%, 39%, 40%, 41%, 42%, 43%, 44%, 45%, 46%, 47%, 48%, 49%, 50%

Upper limit: 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%

The non-structural ingredient may be present in trace amount, but may be present in greater amounts in the range 0.1 % - 25% by weight of total dry ingredients.

The non-structural ingredients may be present in a range (by percent of total dry ingredients) wherein one of the following lower range limits is combined with one of the upper range limits:
Lower limit: 0.0%, 0.05%, 0.1%, 0.5%, 0.75%, 0.8%, 0.9%, 1%, 2%, 2.5%, 3%, 4%, 5%, 7.5%, 10%
Upper limit: 11%, 12%, 13%, 14%, 15%, 16%, 17%, 18%, 19%, 20%, 21%, 22%, 23%, 24%, 25%

The liquid may be selected from water, an aqueous solution, an alcohol or a liquified gas, optionally oxygen or nitrogen; a gas in solid form is also added with the one or more liquids to the solid ingredients; preferably the solid gas is carbon dioxide. Liquid ingredients may also include, for example, flavourings in aqueous solution, ingredients in syrup form (e.g. liquid glucose). Combinations of the various liquids are possible. The preferred alcohol is ethanol which may be added as an ingredient in the form of absolute ethanol, or in the form of an alcoholic beverage, e.g. spirit or wine. Advantageously, the presence of ethanol as a liquid ingredient serves as a preservative.

The non-structural ingredient may comprise one or more of a dietary supplement, a nutriceutical, a probiotic, a pharmaceutical, a flavouring, a colouring and a preservative.

A temperature-sensitive ingredient is preferably one which has a threshold temperature at a temperature in the range 10 °C to 75 °C, below which threshold temperature it remains substantially unaltered in respect of one or more of chemical structure, flavour, biological activity, pharmacological activity and nutritional value. The temperature sensitive ingredient may be added before, during or after mixing of the other ingredients with the liquid when forming the dough or paste.

For example, and without limitation, one or more of the following non-structural ingredients may be included: dried coffee extract, lemon flavouring, vanilla flavouring, chocolate flavouring, cheese flavouring, meat flavouring, pepper, potassium chloride, sodium chloride, vitamins, minerals, herbs & herb extracts, pharmaceutical drug substances, e.g. powders and liquids (pain relievers, allergy relief, hormones, etc.), veterinary medicines.

The dietary supplement may be selected from one or more of a vitamin, a mineral, fibre, a plant extract, a fatty acid and an amino acid, or derivatives thereof.

The nutriceutical may be selected from one or more of antioxidants, soluble dietary fiber, a plant extract, and a fatty acid, or derivatives thereof.

The pharmaceutical may be selected from one or more of a small molecule, a protein and a peptide.

The ingredients may further comprise a modifier of the matrix (and thereby modifier of the matrix-forming ingredient) selected from the group consisting of one or more of a milk powder, a sugar, a flour, a bran, a starch, a seed, a ground seed, a ground pulse, a ground bean, a ground pea, a fat and an oil. The matrix modifying ingredient can be used to alter the size of the air pockets and/or the fragility (brittleness) of the expanded food product.

In addition or alternatively to the non-structural ingredients noted above, the ingredients may further comprise one or more of a flavouring, a colouring and a preservative.

The flavouring may be selected from one or more of an artificial flavouring, a plant extract, a dried plant powder, a salt, a sweetener and a sugar.

The combining of ingredients together may comprise one or more of mixing, blending, pressing and extrusion. Ingredients can be mixed in a food mixer, such as a Hobart food processor or a Z Blade mixer. Advantageously the processes allow an ambient temperature to be maintained for the dough or paste. Heat generated by vigorous mixing or kneeding of stiff dough or paste and which leads to temperatures exceeding 75 °C is to be avoided.

In the method of the invention, the pieces of dough or paste are treated so that they attain a temperature not exceeding 75 °C, at which temperature evaporation of liquid and/or transition from solid to gas is sufficient to expand the pieces when subjected to at least a partial vacuum. In preferred embodiments, the dough or paste attains a temperature not exceeding a temperature selected from one of the following: 15 °C, 16°C, 17 °C, 18 °C, 19 °C, 20 °C, 21 °C, 22 °C, 23 °C, 24 °C, 25 °C, 26 °C, 27 °C, 28 °C, 29 °C, 30°C, 31°C, 32°C, 33 °C, 34°C, 35°C, 36°C, 37 °C, 38 °C, 39 °C, 40 °C, 41°C, 42 °C, 43 °C, 44°C, 45 °C, 46 °C, 47 °C, 48 °C, 49 °C, 50°C, 51°C, 52°C, 53 °C, 54 °C, 55 °C, 56 °C, 57 °C, 58 °C, 59 °C, 60 °C, 61 °C, 62 °C, 63 °C, 64 °C, 65 °C, 66 °C, 67 °C, 68 °C, 69 °C, 70 °C, 71 °C, 72 °C, 73 °C, and 74°C. In order to achieve this, depending on the temperature of the dough or paste immediately it is formed on combining of the ingredients, the treating of the pieces of dough or paste may comprise warming or cooling for a period of time.

The warming may comprise exposure of the pieces to one or more of microwave heating, convective heating, conductive heating, infrared heating and dielectric heating.

When pieces of dough or paste are exposed to higher temperatures, e.g. in the range 40°C - 75 °C for a suitable period, this serves to adjust the moisture content of the shapes prior to the next step. Advantageously, such higher temperatures in this step leads to a material quality differential that continues through to the expansion step. By adjusting the moisture content in the zone of the shaped mixture, a surface layer can be formed, which may be of lesser or greater water content than the interior portion of the shape. The temperature and time allowed for this conditioning step may be used to adjust the relative thickness and resilience of the surface layer as compared to the remainder of the mixture in the shape. The temperature of the dough or paste during this conditioning step is preferably below the temperature of the dough or paste in the step of vacuum expansion.

The treating of the pieces may comprise allowing them to rest for a period of time at the desired temperature sufficient for the desired temperature to be attained by the pieces.

The combining of ingredients in step (a) of the method of the invention resulting in a dough or paste may be carried out at a temperature not exceeding a temperature selected from: 74 °C, 73 °C, 72°C, 71 °C, 70 °C, 69°C, 68 °C, 67 °C, 66 °C, 65°C, 64 °C, 63°C, 62°C, 61 °C, 60 °C, 59 °C, 58 °C, 57 °C, 56 °C, 55 °C, 54 °C, 53 °C, 52 °C, 51 °C, 50 °C, 49°C, 48 °C, 47°C, 46 °C, 45 °C, 44 °C, 43°C, 42 °C, 41 °C, 40 °C, 39 °C, 38°C, 37°C, 36°C, 35°C, 34°C, 33°C, 32 °C, 31 °C, 30 °C, 29 °C, 28 °C, 27 °C, 26 °C, 25°C, 24°C, 23 °C, 22 °C, 21°C, 20 °C, 19°C, 18°C, 17°C, 16°C, 15°C, 14 °C, 13°C, 12°C, 11 °C, 10°C, 9 °C, 8 °C, 7 °C, 6 °C, 5°C, 4°C, 3 °C, 2 °C and 1 °C. In the aforementioned, the temperatures are also the temperature of the resultant dough or paste and wherein the minimum temperature is preferably 0 °C.

The step (a) of combining ingredients in accordance with the method of the invention is preferably carried out at an "ambient temperature", i.e. the temperature of the room where processing manufacture is taking place, e.g. a temperature in the range of about 10°C to 40°C, preferably in the range of about 16°C to 28°C, more preferably in a range of about 18°C to 24°C; also preferred is a temperature in the range 10 °C - 25 °C. The rate and time of mixing is preferably controlled in order to keep the temperature of the mixture within the desired limits.

The combining of any dry ingredients making up the dough or paste, such as powders or particulate materials, is carried out prior to the addition of liquid or any damp, aqueous or liquid ingredients. The powder may be blended in a commercially available blender to form a dry mixture, and this is preferably substantially homogeneous. Other ingredients which are not dry or in powder form can then be mixed with the blended dry ingredients. The further mixing of the preferably substantially homogenous powder mixture with any damp, aqueous or liquid ingredients may be carried out in the same or a different blender or vessel as the blending of the powder. After sufficient mixing of ingredients the resultant mixture is preferably in the form of a moist or crumbly powder mixture or partially agglomerated powder mixture that is compressible, extrudable or mouldable.

Some dry ingredients can be substituted for equivalents in different form. For example, powders can be substituted by syrups, such as in the case of glucose.

The exposing of pieces of dough or paste to the at least partial vacuum may be at a temperature not exceeding a temperature selected from: 74°C, 73 °C, 72 °C, 71 °C, 70 °C, 69 °C, 68 °C, 67 °C, 66 °C, 65 °C, 64 °C, 63 °C, 62 °C, 61 °C, 60 °C, 59 °C, 58 °C, 57 °C, 56 °C, 55°C, 54 °C, 53 °C, 52 °C, 51°C, 50 °C, 49 °C, 48 °C, 47 °C, 46 °C, 45 °C, 44 °C 43 °C, 42 °C, 41 °C, 40 °C, 39 °C, 38°C 37 °C, 36 °C, 35 °C, 34 °C, 33 °C, 32 °C, 31 °C, 30°C, 29 °C, 28 °C, 27 °C, 26 °C, 25 °C, 24 °C, 23 °C, 22 °C, 21 °C, 20 °C, 19 °C, 18°C, 17°C, 16 °C, 15 °C, 14 °C, 13 °C, 12 °C, 11 °C, 10°C, 9°C, 8 °C, 7 °C, 6°C, 5 °C, 4°C, 3 °C, 2 °C and 1°C. The minimum temperature is preferably 0°C.

The exposing of pieces of dough or paste to a vacuum may be within a temperature range in which at a lower limit is selected from one of: 0°C, 1°C, 2°C, 3°C, 4°C, 5 °C, 6 °C, 7 °C, 8°C, 9°C or 10°C; and where an upper limit is selected from 11°C, 12 °C, 13°C, 14°C, 15°C*,* 16°C, 17°C, 18°C, 19°C, 20°C, 21°C, 22°C, 23°C, 24 °C, 25 °C, 26°C, 27°C, 28°C, 29°C, 30°C, 31°C, 32°C, 33°C, 34°C, 35°C, 36°C, 37°C, 38 °C, 39 °C, 40 °C, 41°C, 42 °C, 43 °C, 44 °C, 45 °C, 46 °C, 47 °C, 48 °C, 49 °C, 50 °C, 51 °C, 52°C, 53°C, 54°C, 55 °C, 56°C, 57°C, 58°C, 59°C, 60°C, 61 °C, 62°C, 63°C, 64 °C, 65°C, 66°C, 67°C, 68 °C, 69°C, 70 °C, 71°C, 72 °C, 73 °C, 74 °C and 75°C.

The exposure of pieces to the at least partial vacuum is preferably at a temperature in the range 15°C - 75 °C ; more preferably in the range 15°C - 60 °C; even more preferably in the range 15°C - 55 °C; most preferably a temperature in the range 35 °C - 55°C.

The shapes are expanded preferably under at least a partial vacuum. A vacuum of less than 13.5kPa (about 100mm Hg mercury) is preferred, optionally in the range 1.30kPa - 12kPa (about 10mm Hg - about 90mm Hg), 2.6kPa - 10.7kPa (about 20mm Hg - about 80mm Hg) or 3.3kPa - 6.7 kPa (about 25mm Hg - about 50mm Hg. The vacuum may be introduced gradually and removed gradually during the expansion process. The vacuum may be introduced and present for only a portion of the expansion process, and is preferably present for more than half of the expansion process step time. In one embodiment, the vacuum is present for all of the time of the expansion step. The possibility exists to have a continuous change in the vacuum or a series of discrete changes in the vacuum during the expansion step. The vacuum serves to allow a lower temperature vaporisation of aqueous and/or volatile components of the mixture than would occur in the absence of any vacuum. The selection of the amount of vacuum to be used over time is a function of the degree of vaporisation that is desired and may have an effect on the precise structure of the product. The resulting structure may be, for example, porous, brittle, crunchy, honeycombed, crispy, or any combination thereof.

The forming of the mixture into shapes may, in one embodiment, be pelletization of the mixture. This can be done using a pelletizer or drop roller. In another embodiment, the mixture may be extruded and cut into pellets with a blade.

Optionally, after pelletizing, the pellets may be kept at a temperature in the range of minus (-) 10°C to ambient (as previously defined) in order to improve ease and convenience and handling of the pellets or shapes.

The mixture can be formed into any desired shape by any well known process such as rolling, extruding, chopping, cutting, moulding, stretching or compressing. The size of each individual shape is not critical, but advantageously, the inventors have found that pieces sized in the range of about 0.1 cm³ to 100 cm³, optionally 0.1 cm³ to 1 cm³ provide a useful shape.

For example, the solid can be extruded into any desired shape which can then be further cut or portioned into other desired shapes. The solid can be mouldable and placed in moulds of the desired shape and optionally pressed into the mould. For example, solid bars can be made ultimately yielding variously sized crunchy products. The extrusion or shaping may result in, for instance, pellet, spherical, cylindrical, tablet, bar, or flake shapes. The word "pellet" or "pellets" is used at various points throughout to describe an example of the resulting extruded and shaped mixture during further processing steps; however, any and all of the processing steps of any embodiments of the invention as described or suggested herein may be carried out with any of the resulting forms following extrusion or shaping.

In certain preferred embodiments, the dough or paste is shaped into the form of small spherical pellets. During the expansion step, these pellets are preferably placed in a drum which is rotated in order to tumble the pellets. Advantageously, this assists in maintaining the spherical shape during expansion. The drum is heated to the desired temperature, and the body of the drum evacuated to the desired degree. The combination of heat and vacuum in the drum causes water or other liquid or solidified gas in the mixture to evaporate or sublime. This process causes material expansion in the body of the spherical pellets and creates steam or gas pockets. Once sufficient steam or gas is generated, the structural integrity of the surface layer of the pellets or other shapes is breached, and the steam or gas escapes, in one embodiment leaving a porous structure. The vacuum is released and the product retrieved and preferably allowed to dry and/or cool. The resulting product, in this embodiment a spherical one, preferably has a smooth outer surface and preferably a brittle interior of air pockets.

In one embodiment, expansion of shapes or pellets is carried out in a batch vacuum oven containing trays. The resulting post-expansion pellets in this embodiment are preferably non-spherical, nuggets, bars, or other shapes determined by the mould shapes supported by the batch vacuum oven trays.

Optionally, after the expansion of the shapes, the vacuum can be left in place whilst drying and/or cooling takes place. Advantageously, this creates products which are of low moisture content and which therefore have a longer shelf life. The employment of vacuum after expansion and during any subsequent steps can be used to modify the structure and texture of the products to a finer degree.

In accordance with preferred embodiments of the method of the invention, after combining ingredients, the resulting dough or paste is allowed to rest together for a period of time sufficient for an equilibration of the liquid and the other ingredients to take place. This resting or "conditioning" of the mixture before the expansion step may be carried out by exposing said mixture to a temperature that is lower than the mixing temperature. A typical range of temperature at which the mixture is held for a period of time is 5 °C to 40 °C, preferably 5 °C to 20 °C, more preferably 5 °C to 15 °C, even more preferably 5 °C to 10 °C. In some embodiments, the conditioning involves chilling of the dough or paste at a temperature of between about -10°C to ambient temperature. This conditioning step is preferably carried out at temperature lower than that at which the shaped or moulded pieces of dough or paste are allowed to rest prior to their vacuum expansion. The resting of the ingredient mixture may take place in a sealed container or in a moisture-controlled environment. The resting of the ingredient mixture may be carried out at atmospheric or subatmospheric conditions which do not result in expansion of the mixture due to vacuum induced boiling of water.

The conditioning step prior to shaping dough or paste pieces may take the form of a reduction in the temperature of the mixture (compared to the temperature at which the combining of ingredients was carried out). In this instance, the inventors refer to this stage as a "cold conditioning" step, usually carried out at a temperature ranging between about 5°C and 40°C. In one embodiment, the cooling is carried out using a brine-cooled heat exchanger. Cooling preferably makes the dough amenable to extrusion or shaping, for example, pellet formation or other moulding into desirable shapes and sizes.

Without wishing to be bound by theory, it is believed that the conditioning of the paste or dough shapes achieves two goals: it brings the temperature of the mixture closer to the subsequently applied expansion temperature, and it adjusts the surface moisture content of the mixture. This latter goal can include either drying or adding moisture to the surface of the mixture and preferably creating a surface layer on the surface of the pellet capable of being expanded during the expansion step, much in the way of a balloon.

The liquid content of the dough or paste is preferably in the range 5% - 25% by weight, preferably 8% - 20% by weight, more preferably 4% - 15%. The mixture may have the texture and appearance of a soft dough or stiff gum or putty. Such mixtures may have elastic or inelastic properties.

The expanded food product is preferably dried under at least a partial vacuum and at a temperature of 75°C or less, preferably at a temperature of 60°C or less, more preferably at a temperature of 55°C or less.

The drying of expanded food product under at least a partial vacuum may be at a temperature not exceeding a temperature selected from: 74 °C, 73 °C, 72°C, 71 °C, 70°C, 69°C, 68°C, 67°C, 66°C, 65 °C, 64 °C, 63 °C, 62 °C, 61 °C, 60°C, 59 °C, 5 8°C, 57°C, 56 °C, 55°C, 54 °C, 53 °C, 52 °C, 51 °C, 50 °C, 49 °C, 48 °C, 47 °C, 46 °C, 45 °C, 44 °C, 43 °C, 42 °C, 41 °C, 40 °C, 39°C, 38 °C, 37 °C, 36°C, 35°C, 34 °C, 33 °C, 32 °C, 31 °C, 30°C, 29°C, 28°C, 27°C, 26°C, 25°C, 24°C, 23°C, 22 °C, 21 °C, 20 °C, 19°C, 18 °C, 17 °C, 16°C, 15 °C, 14°C, 13 °C, 12 °C, 11°C, 10 °C, 9°C, 8 °C, 7 °C, 6 °C, 5°C, 4 °C, 3 °C, 2 °C and 1°C. The minimum temperature is preferably 0°C.

The drying of expanded food product under at least a partial vacuum may be at temperature with a temperature range in which at the lower limit is selected from one of 0°C, 1°C, 2 °C, 3 °C, 4 °C, 5°C, 6 °C, 7 °C, 8°C, 9°C or 10 °C; and where independently the upper limit is selected from one of: 11°C, 12°C, 13 °C, 14°C, 15 °C, 16°C, 17°C, 18°C, 19°C, 20°C, 21 °C, 22 °C, 23°C, 24°C, 25°C, 26°C, 27°C, 28 °C, 29°C, 30°C, 31°C, 32°C, 33°C, 34°C, 35°C, 36°C, 37°C, 38 °C, 39 °C, 40 °C, 41°C, 42°C, 43°C, 44°C, 45°C, 46°C, 47°C, 48°C, 49°C, 50°C, 51°C, 52 °C, 53 °C, 54 °C, 55 °C, 56 °C, 57 °C, 58 °C, 59 °C, 60 °C, 61 °C, 62 °C, 63 °C, 64°C 65°C, 66°C, 67°C, 68 °C, 69°C, 70 °C, 71°C, 72 °C, 73°C, 74 °C and 75 °C.

The drying of the expanded mixture or shape is preferably at a temperature in the range 40°C to 75°C. The temperatures used in any drying process step are preferably the same or substantially similar to the temperatures used in the expansion step.

The drying step may be carried out in the vacuum expansion vessel or in a separate vessel. This step can prevent deflation or partial collapsing of the expanded pellets. The pellets may also be cooled, for example with dry cold air blowers, and/or coated, either directly after expansion or after drying.

The expanded food product after drying preferably contains between 0 - 2% moisture by weight; more preferably 0.01 % - 2%, even more preferably 0.1 % - 2%.

In other embodiments of the method of the invention, the steps further comprise the step of cooling the expanded food product. The cooling is preferably under at least a partial vacuum.

In all steps of the method of the invention where at least a partial vacuum is used, the vacuum is in the range 1Pa - 13.4kPa, preferably 0.67kPa - 8kPa (about 5 to about 60 mm Hg).

The internal structure of air pockets is preferably brittle, i.e. giving a "honeycomb" structure.

Methods in accordance with the invention may be run as a batch process or as a continuous process either by using commercially available equipment readily available and well known to a person of skill in the art, or bespoke processing equipment.

At all steps in the process of making the food products of the invention, the temperature is preferably always kept below the temperature which is known to result in deleterious effect to the incorporated additive and/or flavouring ingredients. Such temperatures will be well known to the person of average skill in the art for the additive or flavouring ingredient alone. Because the additive or flavouring ingredient will be present in a complex mixture during the process of making the product, there may be some possibility to allow an increase in temperature above the theoretical limit briefly or to a degree.

Application of the method of the invention and the resulting food products include nutriceuticals, snacks, cereals, sports (energy) foods, body building supplements and foods, health supplements, oral veterinary products, oral pharmaceutical products, or animal feed, including bird or fish food.

Described herein are expanded food products or food products for humans or animals made in accordance with the process of the invention. Preferably, such products are honeycombed and of a brittle and crunchy consistency.

Described herein is an expanded food product made in accordance with the process of the invention having an internal structure of air pockets and comprising:
(i) a structural matrix-forming ingredient selected from dietary fibre and/or hydrolysed wheat flour; and
(ii) moisture in the range 0 - 4% by weight.

Preferably, the dietary fibre is selected from one or more of soluble dietary fibre, prebiotic dietary fibre, polydextrose and soluble corn fibre.

The moisture content may be the range 0% - 3%, 0%-2% or 0%-1%. Optionally the minimum water content may be 0.05%, 0.1% or 0.5% in combination with the upper limits of the previous ranges.

Components (i.e. ingredients) of the expanded food products are as described in relation to the methods of the invention above.

Preferably the internal structure of air pockets is brittle, e.g. a honeycomb, and the density of the air pockets may be controlled by the adjustment of relative quantities of ingredients used to make the dough or paste, and/or by fine adjustment of the temperatures, pressures (vacuum) and timings of the process steps within the ranges described herein.

Pieces of the expanded food product of the method of the invention may have the shape of a sphere, a cylinder, a bar, a tablet, or a flake; or are irregular. Products of the method of the invention may have a weight in the range 0.1g - 100g. Products of the method of the invention may have a volume in the range 0.1 cm³ to 100 cm³, optionally 0.1cm³ to 10cm³ or 0.1cm³ to 1cm³. Products of the method of the invention may have at least a partial surface coating.

Products of the method of the invention preferably have a crunchy, porous texture, and may be of various shapes including, for example, spheres or balls, cylinders, bars, tablets, or flakes, and may have for example, a sweet, savoury, tart, sour bitter or salty taste. Umani may be used to flavour the product.

The expanded food product may be a nutriceutical, a snack, a cereal, a sports food, an energy food, a body building supplement, a food or health supplement, an oral veterinary product, an oral pharmaceutical product, or an animal feed.

Coatings, for example sugar or chocolate, may be selected to affect taste, texture, or a combination thereof, and to act as a moisture barrier, extending the shelf life of the final product. Preferably, the shelf life of the end food product is greater than about 1 month, greater than about 3 months, between about 6 and 9 months, or greater than about 9 months.

The invention will now be described in detail and with reference to specific examples.

Examples of commercially available matrix-forming ingredients are:
Prebiotic dietary fibre: NUTRIOSE ® produced Roquette
Polydextrose: STA-LITE ® produced by Tate & Lyle
Soluble corn fibre: PROMITOR ® (trademark) produced by Tate & Lyle FARIGEL ® WHEAT LV PRODUCED BY WestHove/Limagrain Cereales
Whey protein isolate: ISOLAC (tradename) produced by Carbery
Whey protein concentrate: CARBOLAC
Hydrolysed whey protein: OPTIPREP
Whey Protein Isolate: NUTRISPORT ® 90+ protein

The modifying ingredients are selected to adjust the structure and texture of the final product. For example milk powders, including full cream milk powder (preferably between about 0 and 20 % of dry weight ingredients) act to soften the final product. Skim milk powder (preferably between about 0 and 30 % dry weight ingredients) will act to make the final product more soft or friable. Sugars, including glucose powder (preferably between about 0 and 40 % dry weight ingredients) or syrup, act to soften the final product giving it greater chewiness. Fructose powder (between about 0 and 40 % dry weight ingredients), or syrup will serve as a softener often imparting chewiness and stickiness to the final product.

Cereal ingredients, including wheat flour, corn starch, oat flour, wheat bran, barley flour, malted barley will each serve as bulking and retention agents.

Seed and pulse-based ingredients, including flax seed, ground lentils, ground rice, ground peas and beans, ground sunflower seeds will serve as bulking & retention agents.

Fats and oils will serve as softening agents. For example, dairy fat (preferably between about 0 and 10 % weight of dry weight ingredients) and vegetable fat (preferably between about 0 and 10 % weight of dry weight ingredients).

Concentrated protein powder, bulking agents, dietary fibre, sugar syrups or extracts, flavoured milk powders, or any combinations thereof, may also be included, alone or in combination as modifiers of the essential matrix ingredients.

The following is a non-exhaustive list of the various flavouring ingredients which may be used in accordance with the invention:
Vanilla extract
Lemon oil/concentrated extract
Powdered ginger
Fruit flavourings (including strawberry, raspberry, lime, orange, blackcurrant, grapefruit, peach, cherry, apricot, apple, pear, banana)
Coffee extract
Chocolate and chocolate flavouring
Butter flavouring
Savoury flavourings (including prawn, smokey bacon, salt & vinegar, beef, cheese, cheese & onion, onion, garlic)
Spices (including peppers, paprika, chilli, cumin, cinnamon, clove, mustard, cardamom, saffron, turmeric)
Salts (sodium chloride, potassium chloride)
Herbs (including sage, parsley, rosemary, coriander, mint, basil, dill, taragon, thyme) Sweeteners (including sucrose and above-named sugars, erythritol, saccharin, sucralose, aspartame, stevia, neotame, sorbitol, xylitol, polyols).

The following is a non-exhaustive list of the various nutritional or medicinal ingredients which may be used in accordance with the invention:
Vitamins (including A, B1, B2, B6, B9, B12, C, D, E, K)
Minerals (including Iron, Calcium, Magnesium, Zinc, Selenium, Iodine, Phophorus) Lutein
Creatine
Zeaxanthin
Resveratrol
Resistant starch
Glucosamine
Glutamine
Beta-carotene
Tea and fruit extracts (including green tea and blackcurrent extract)
Omega 3 (EPA, DHA, ALA)
Painkillers (including aspirin, paracetamol, ibuprofen)
Allergy and asthma medicines (including anti-histamines, beclomethasone) Antibiotics
Anti-inflammatory medicines
Diuretics
Hormone supplements
Anti-oxidant protection medicines
Arthritis and rheumatism medicines
Urinary infection medicines
Detoxification products
Digestive system medicines
Diabetes medicines
De-worming medicines

### EXAMPLES

There will now be described in detail the process steps used in the series of specific examples of expanded food products in accordance with the invention.

### 1. Dry ingredient dispensing

Dry ingredients are assembled. The equipment and procedures for dispensing appropriate amounts of ingredients for mixing together are well established and well known to a person of average skill in the art. Equipment includes weighing scales and loss-in-weight feeders, for example.

### 2. Liquid ingredient dispensing

Liquid ingredients are assembled. The equipment and procedures for dispensing appropriate amounts of liquid ingredients for mixing with other liquids and/or solids are well established and well known to a person of average skill in the art. Equipment includes measuring cylinders and liquid dispensing pumps, for example.

### 3. Powder blending

The mixing of dry ingredients makes use of well-established process technology and equipment, such as batch powder mixers and continuous powder mixers.

### 4. Powder & liquid blending

This is achieved by adding liquids slowly to pre-mixed powders in a conventional powder mixer/blender to form a crumbly, slightly agglomerated blend of powders and liquids. At this stage, although the mixture might appear relatively homogeneous, the liquids are not yet uniformly dispersed throughout the powder at a microscopic, submicroscopic or molecular level. An important characteristic of this process step is that very little heat is generated through friction during the mixing process, so the temperature of the mixture remains close to ambient (i.e. room temperature) which may be in the region of 10 °C to 25 °C).

### 5. Compaction

The mixture of solid and liquid ingredients is compressed at ambient temperature by batch (simple pressing or moulding) or continuous (e.g. gentle extrusion) compaction, such that very little heat is generated. This results in a malleable 'dough', the stiffness of which typically ranges from that of a soft bread or biscuit dough (viscosity in the region of 1000Pa.s) to that of chewing gum, chewable toffee or window putty (viscosity in the region of 100,00OPa.s. Again, the temperature of the dough would remain close to ambient (in the region of 10 °C to 25 °C). A key feature of the process is that kneading of the dough is avoided to minimise heating as a result of friction. Similarly, extrusion processes that would cause the dough temperature to rise significantly are avoided. Dough temperatures are kept low to avoid heat degradation or destruction of any heat-sensitive ingredients, and to avoid unwanted flavour development within the dough.

### 6. Conditioning

The dough is allowed to sit to "condition" in a sealed container to avoid moisture uptake (given that some of the ingredients are hygroscopic), surface drying and oxidation. This step may varying in duration (typically between 1 minute and 1 hour), to allow the water (or other evaporation expansion agents) in the dough to be dispersed and/or absorbed more uniformly. Uniform dispersion of the expansion agent(s) ensures best uniformity of expansion in step 9. However, in some situations, a non-uniformity of expansion may be desirable to achieve a desired end-product textural character, in which case limited or no conditioning may be required.

### 7. Dough Shaping

Dough is formed into pieces of the desired size and shape. Examples include round pellets which are formed using drop rollers, tablets which are formed in tablet-making machines; cylindrical pellets which are produced by cold-extruding the dough through round holes in a die to form thin strands of circular cross-section and then cutting these into short lengths to form cylinders, and flat shapes which are formed by rolling the dough into flat sheets and then creating the desired shapes with knives or other cutting devices.

In some embodiments, the compaction step 5 can be omitted and the agglomerated powder pressed directly into shapes.

### 8. Pre-heating and further conditioning

Pre-heating is carried out to bring the dough pieces to, or close to the temperature at which the expansion (step 9) is to be carried out. This is achieved by various heat transfer processes, including radiant heating, conduction and/or convection, microwave or dielectric heating. The period of heat transfer process may take a range of time, typically from a few seconds to 30 minutes. Further conditioning occurs during this step.

In some examples, the conditioning step 6 is omitted and any conditioning is left to the pre-heating (conditioning) step after the dough has been shaped into the desired pieces.

### 9. Expansion

Expansion is carried out by boiling the water and/or other expansion agents under reduced pressure by applying a vacuum. The expansion occurs partly as a result of the vapour or gas initially being trapped within the dough. In many cases a surface layer of low porosity is formed which assists the expansion by slowing down or preventing the release of the vapour or gas. As expansion progresses, vapour or gas bubbles are formed which lead to a cellular, honeycomb-like structure being created, contained within a porous or semi-porous surface layer. As the vapour or gas is released, the dough dries and hardens, thereby creating a stable cellular or honeycomb-like structure, which can range from being hard and crunchy to soft and chewy depending on the selection of ingredients.

Expansion is achieved typically between 15 °C and 75 °C, but commonly a temperature is used in the range 35 °C to 55 °C, depending partly on the vaccuum pressure applied. Vacuum pressures are typically in a range between 0.6kPa and 13.4kPa (about 5 mm Hg and about 100 mmHg), but commonly between 1.3kPa and 8kPa (about 10 mmHg and about 60 mmHg. Expansion occurs over a period of typically one to 10 minutes, depending partly on how quickly the vacuum is applied, and partly on the amount of water and/or expansion agent contained within the dough. Water and/or expansion agent contents are typically in the range of 5% to 25% by weight, but commonly between 8% to 20%. Low expansion temperatures are used (1) to prevent heat degradation or destruction of any heat sensitive ingredients, (2) to prevent unwanted flavour development within the product, and (3) in certain situations to avoid or inhibit formation of acrylamides.

### 10. Drying

Drying of the expanded cellular, or honeycomb-type structured products can be continued after expansion to further stabilise and harden the cell walls and surfaces of the product, typically at similar pressures and temperatures as used in step 9 expansion. Post-expansion drying times are typically between 5 and 30 minutes, partly determined by the weight of the individual product pieces. The moisture contents of the dried products are typically between 0% and 4% moisture; optionally 0% and 2%.

### 10. Cooling

Where it is desired to stabilise the expanded products more quickly, then they can be cooled. This cooling can be done before or after the vacuum is released. Cooling can be performed quickly, by chilling the product in a sealed container to prevent moisture being absorbed. Cooling times are typically between 1 and 10 minutes, and final cold product temperatures are typically between 0°C and 25 °C.

### Example 1 - a crunchy snackfood

Hydrolysed wheat flour (40% weight) is blended with glucose powder (25% weight), fructose powder (25% weight), and skim milk powder (10% weight) and trace amounts of vitamins and other desirable nutrients. Vanilla extract is next added to the powder mixture in an aqueous solution in an amount of 5% of the resultant composition's weight. The moisture content of this composition at this point is 9%. The composition is cold conditioned to 5 °C for ease of pellet formation. The composition is next formed into pellets and chilled to 0 °C for ease of handling of the pellets. The pellets next undergo pre-expansion conditioning, which comprises heating them to 50 °C. The pellets are next tumbled gently in a rotating partial-vacuum cylinder maintaining a pressure of 2.67kPa (about 20 mm Hg) and heated to 50 °C so that the pellets may expand. The pellets are next dried in the same vessel and at the same pressure and temperature at which expansion occurred. The resultant food product is a ball-shaped, honeycombed, sweet crunchy snack, containing vitamins and nutrients.

### Example 2 - high protein snack for body builders

Polydextrose (40% weight) is blended with concentrated protein powder (50% weight), and fructose (10% weight). Vanilla extract is next added to this mixture in an aqueous solution in an amount of 5% of the resultant composition's weight. The moisture content of the composition at this point is 20%. The composition is shaped into bars, which next undergoes pre-expansion conditioning by heating to 40 °C. The bars are next placed into the mould shapes of a batch vacuum tray where they undergo expansion at a pressure of 3.3 kPa (about 25 mm Hg) and a temperature of 40 °C. The bars are then dried at a pressure of 3.3kPa (about 25 mm Hg) and a temperature of 35 °C. The resultant food product is a sports nutrient bar tailored for body builders.

### Example 3 - sweet vanilla crunchy snacks with vitamin C

### Ingredients:

Hydrolysed wheat flour (40% by weight)
Glucose powder (25% by weight)
Fructose powder (25% by weight)
Skim milk powder (10% by weight)
Vanilla extract (minor - added in aqueous solution)
Vitamin C powder (minor - to achieve RDA in 30g portion).

Dough moisture content: 9% by weight
Conditioning time: 10 minutes
Dough pieces: 1g weight; spherical
Pre-heating and conditioning: to 40 °C ; 5 minutes
Expansion: 40 °C ; 10 minutes, 1.3kPa (about 10mm Hg)
Drying: 40 °C ; 20 minutes, 1.3kPa (about 10mm Hg)
Cooling: 5 °C ; 10 minutes; atmospheric pressure
Final product: Dry, crunchy, spherical pieces

### Example 4 -sweet ginger biscuits with fish-oil omega 3

### Ingredients:

Soluble dietary fibre (45% by weight)
Hydrolysed wheat flour (35% by weight)
Fructose powder (10% by weight)
Skim milk powder (10% by weight)
Ginger powder (minor)
Vanilla extract (minor)
Concentrated odorless fish-oil based omega 3 (typical recommended daily dose in 40g portion)

Dough moisture content: 15% (by weight)
Conditioning time: 15 minutes
Dough pieces: 2g weight; spherical
Pre-heating and conditioning: to 45 °C for 4 minutes
Expansion: 40°C for 15 minutes at 1.3kPa (about 10mm Hg)
Drying: 40 °C for 30 minutes at 1.3kPa (about 10mm Hg
Cooling: 5 °C for 15 minutes at atmospheric pressure
Final product: 'Macaroon'-shaped biscuits, crunchy with slight chewiness, pleasant ginger flavour, with no hint of fish flavour. The fish oil omega 3 would normally oxidise in conventional baking, causing a strong fishy flavour to develop that many consumers would find unpleasant. In this example, the fishy flavour does not develop from the fish oil.

### Example 5 - savoury protein-rich sports snack bar

### Ingredients:

Whey protein isolate (55% by weight)
Polydextrose (45% by weight)
Cheese & onion flavouring (minor)
Creatine (minor)

Dough moisture content: 18% (by weight)
Conditioning time: 10 minutes
Dough pieces: 15g weight; rectangular bar
Pre-heating and conditioning: to 40°C for 5 minutes (within an open-top rectangular mould)
Expansion: 40 °C for 5 minutes at 0.67kPa (about 5mm Hg)
Drying: 40 °C for 20 minutes at (0.67kPa (about 5mm Hg)
Cooling: 0 °C for 10 minutes at atmospheric pressure
Final product: rectangular, dry, crunchy snack bar.

### Example 6 - sweet crunchy breakfast cereal with added vitamins and minerals

### Ingredients:

Soluble corn fibre (45% by weight)
Hydrolysed wheat flour (30% by weight)
Fructose (10% by weight)
Full cream milk powder (5% by weight)
Skim milk powder (5% by weight)
Wheat bran (5% by weight)
Vanilla extract (minor)
Vitamin and mineral pre-mix (minor)
Glucosamine (minor)

Dough moisture content: 9% by weight
Conditioning time: 20 minutes
Dough pieces: 0.5g cylinders (height = diameter)
Pre-heating and conditioning: to 45 °C for 5 minutes
Expansion: 45 °C for 8 minutes at 0.67kPa (about 5mm Hg) in a rotating, cylindrical, vacuum vessel
Drying: 45 °C for 5 minutes at 0.67kPa (about 5mm Hg)
Cooling: 4 °C for 5 minutes at atmospheric pressure
Final product: dry, crunchy breakfast cereal, consisting of approximately spherical pieces

### Example 7 - coffee-flavoured nutritional biscuits

### Ingredients:

Prebiotic soluble dietary fibre (40% by weight)
Hydrolysed wheat flour (30% by weight)
Glucose powder (15% by weight)
Skim milk powder (10% by weight)
Wheat bran (5% by weight)
Coffee extract (minor)
Vanilla extract (minor)
Concentrated odourless fish-oil based omega 3 (typical recommended daily dose in 40g portion)
Lutein (minor)
Vitamin and mineral premix (minor).

Dough moisture content: 12% by weight
Conditioning time: 20 minutes
Dough pieces: 4g weight; circular discs
Pre-heating and conditioning: to 40 °C for 10 minutes
Expansion: 40 °C for 10 minutes, 1.3kPa. (about 10mm Hg)
Drying: 40°C for 30 minutes at 1.3kPa (about 10mm Hg)
Cooling: 5 °C for 15 minutes at 1.3 kPa (about 10mm Hg)
Final product: circular biscuits, crunchy with slight chewiness.

### Example 8 - beef-flavoured crunchy petfood snacks with vitamins and minerals

### Ingredients:

Soluble corn fibre (50% by weight)
Hydrolysed wheat flour (30% by weight)
Whey protein concentrate (10% by weight)
Wheat bran (5% by weight)
Ground rice (5% by weight)
Beef flavouring (minor)
Vitamin and mineral pre-mix (minor)

Dough moisture content: 10% by weight
Conditioning time: 10 minutes
Dough pieces: 0.25g, 0.5g and 1g cylinders (height = diameter)
Pre-heating and conditioning: to 50 °C for 5 minutes
Expansion: 50 °C for 8 minutes at 1.3kPa (about 10 mm Hg), (in a rotating, cylindrical, vacuum vessel)
Drying: 50 °C for 5 minutes at 1.3kPa (about 10 mm Hg
Cooling: 0°C for 5 minutes at atmospheric pressure
Final product: dry, crunchy snacks, consisting of approximately spherical pieces

### Example 9 - sweet lemon-flavoured crunchies with paracetamol, vitamin C and vitamin and mineral premix

### Ingredients:

Hydrolysed wheat flour (40% by weight)
Polydextrose (25% by weight)
Fructose powder (25% by weight)
Skim milk powder (10% by weight)
Lemon-oil extract (minor)
Paracetamol (minor - to achieve recommended dose in 20g portion
Vitamin C powder (minor - to achieve RDA in 20g portion)
Mineral and vitamin premix (minor).

Dough moisture content: 8% by weight.
Conditioning time: 15 minutes
Dough pieces: 1g weight; spherical
Pre-heating and conditioning: to 40 °C for 5 minutes
Expansion: 40 °C for 15 minutes at 0.67kPa (about 5mm Hg)
Drying: 40 °C for 20 minutes at 0.67kPa (about 5mm Hg)
Cooling: 0 °C for 10 minutes at atmospheric pressure
Final product: dry, crunchy, spherical pain relievers and nutritional supplements.

## Claims

1. A method of producing an expanded food product having an internal structure of air pockets comprising:
(a) combining together ingredients comprising a matrix- forming ingredient selected from dietary fibre and/or hydrolysed wheat flour;
(b) adding one or more liquids to the ingredients to produce a dough, paste or moist powder at temperature not exceeding 75 °C;
(c) forming the dough, paste or moist powder into individual pieces;
(d) treating the pieces so that they attain a temperature not exceeding 75 °C, at which temperature evaporation of liquid and/or transition from solid to gas is sufficient to expand the pieces when subjected to at least a partial vacuum; and
(e) exposing the pieces to at least a partial vacuum and at a temperature not exceeding 75 °C such that liquid evaporates causing the matrix to expand to form the food product having an internal structure of air pockets.

2. A method as claimed in claim 1, wherein the ingredients further comprise a non-structural ingredient; preferably a temperature-sensitive, non-structural ingredient; more preferably:
(1) wherein the non-structural ingredient comprises one or more of a dietary supplement, a nutriceutical, a probiotic, a pharmaceutical, a flavouring, a colouring and a preservative; and/or
(2) wherein the temperature sensitive ingredient has a threshold temperature at a temperature in the range 10 °C to 75 °C, below which threshold temperature it remains substantially unaltered in respect of one or more of chemical structure, flavour, biological activity, and pharmacological activity.

3. A method as claimed in claim 1 or claim 2, wherein
(1) the dietary supplement is selected from one or more of a vitamin, a mineral, fibre, a plant extract, a fatty acid and an amino acid, or derivatives thereof;
(2) the nutriceutical is selected from one or more of antioxidants, soluble dietary fiber, a plant extract, and a fatty acid, or derivatives thereof; or
(3) the pharmaceutical is selected from one or more of a small molecule, a protein and a peptide.

4. A method as claimed in any preceding claim, wherein the dietary fibre is selected from one or more of soluble dietary fibre, prebiotic dietary fibre, polydextrose and soluble corn fibre.

5. A method as claimed in any preceding claim, wherein the matrix- forming ingredient further comprises whey protein isolate and/or whey protein concentrate.

6. A method as claimed in any preceding claim, wherein the one or more matrix-forming ingredients are present in the range 30% - 100 % by weight of total ingredients when excluding the one or more liquid; preferably 40% - 100% by weight of the said total ingredients.

7. A method as claimed in any preceding claim, wherein (a) the liquid is selected from water, an aqueous solution, an alcohol or a liquified gas, optionally oxygen or nitrogen; and (b) a gas in solid form is also added with the one or more liquids to the solid ingredients, preferably the solid gas is carbon dioxide.

8. A method as claimed in any preceding claim, wherein the ingredients further comprise
a modifier of the matrix selected from the group consisting of one or more of a milk powder, a sugar, a flour, a bran, a starch, a seed, a ground seed, a ground pulse, a ground bean, a ground pea, a fat and an oil.

9. A method as claimed in any preceding claim, wherein the ingredients further comprise one or more of a flavouring, a colouring and a preservative; optionally wherein the flavouring is selected from one or more of an artificial flavouring, a plant extract, a dried plant powder, a salt, a sweetener and a sugar.

10. A method as claimed in any preceding claim, wherein the treating of the pieces comprises warming or cooling for a period of time; optionally wherein the warming comprises exposure of the pieces to one or more of microwave heating, convective heating, conductive heating, infrared heating and dielectric heating.

11. A method as claimed in any preceding claim, wherein the treating of the pieces allows them to rest for a period of time at the desired temperature sufficient for the desired temperature to be attained by the pieces.

12. A method as claimed in any preceding claim, wherein step (a) is carried out at an ambient temperature, e.g. a temperature in the range 10°C - 25 °C.

13. A method as claimed in any preceding claim, wherein the exposure of pieces to the at least partial vacuum is at a temperature in the range 15°C - 75 °C; preferably in the range 15°C - 60 °C; more preferably in the range 15°C - 55 °C; even more preferably a temperature in the range 35°C - 55 °C.

14. A method as claimed in any preceding claim, wherein prior to forming into pieces, the combined ingredients are allowed to rest together for a period of time sufficient for an equilibration of the liquid and the other ingredients to take place; preferably wherein the resting of the ingredient mixture takes place in a sealed container or in a moisture-controlled environment.

15. A method as claimed in any preceding claim, wherein
(1) the expanded food product is dried under at least a partial vacuum and at a temperature not exceeding 75 °C, preferably at a temperature not exceeding 60 °C, more preferably at a temperature not exceeding 55 °C or less; and/or
(2) further comprising the step of cooling the expanded food product; preferably wherein the cooling is under at least a partial vacuum; and/or
(3) the at least partial vacuum is in the range 0.13kPa to 13.4kPa, preferably 0.67kPa to 8kPa.

## Patentansprüche

1. Verfahren zum Erzeugen eines erweiterten Nahrungsmittels mit einer Innenstruktur aus Lufttaschen, wobei das Verfahren folgendes umfasst:
(a) das Zusammenführen von Inhaltsstoffen, die einen matrixbildenden Inhaltsstoff umfassen, der ausgewählt wird aus Ballaststoffen und/oder hydrolysiertem Weizenmehl;
(b) das Hinzufügen von einer oder mehreren Flüssigkeiten zu den Inhaltsstoffen, so dass ein Teig, eine Paste oder ein feuchtes Pulver auf einer Temperatur erzeugt wird, die 75 °C nicht überschreitet;
(c) das Formen des Teigs, der Paste oder des feuchten Pulvers in einzelne Stücke;
(d) das Behandeln der Stücke, so dass diese eine Temperatur erreichen, die 75 °C nicht überschreitet, wobei die temperaturbedingte Verdampfung von Flüssigkeit und/oder der Übergang von der Feststoffphase in die Gasphase ausreicht, um die Stücke zu erweitern, wenn diese zumindest einem teilweisen Vakuum ausgesetzt werden; und
(e) das Aussetzen der Stücke zumindest einem teilweisen Vakuum und auf einer Temperatur, die 75 °C nicht überschreitet, so dass Flüssigkeit verdunstet, was bewirkt, dass die Matrix expandiert, so dass das Nahrungsmittel mit einer Innenstruktur aus Lufttaschen gebildet wird.

2. Verfahren nach Anspruch 1, wobei die Inhaltsstoffe ferner einen nichttragenden Inhaltsstoff umfassen; vorzugsweise einen temperaturempfindlichen, nichttragenden Inhaltsstoff; wobei folgendes darüber hinaus bevorzugt wird:
(1) wobei der nichttragende Inhaltsstoff einen oder mehrere der folgenden Stoffe umfasst: ein Nahrungsergänzungsmittel, ein Nutriceutical, ein Probiotikum, ein Arzneimittel, einen Geschmacksstoff, einen Farbstoff und ein Konservierungsmittel; und/oder
(2) wobei der temperaturempfindliche Inhaltsstoff eine Schwellentemperatur auf einer Temperatur im Bereich von 10 °C bis 75 °C aufweist, wobei der Inhaltsstoff unterhalb dieser Schwellentemperatur im Wesentlichen unverändert bleibt in Bezug auf einen oder mehrere der folgenden Aspekte: die chemische Struktur, den Geschmack, die biologische Aktivität und die pharmakologische Aktivität.

3. Verfahren nach Anspruch 1 oder Anspruch 2, wobei:
(1) das Nahrungsergänzungsmittel aus einem oder mehreren der folgenden Stoffe ausgewählt wird: einem Vitamin, einem Mineralstoff, einem Ballaststoff, einem Pflanzenextrakt, einer Fettsäure und einer Aminosäure oder Derivaten dieser;
(2) das Nutriceutical ausgewählt wird aus einem oder mehreren der folgenden Stoff: Antioxidationsmitteln, löslichem Ballaststoff, einem Pflanzenextrakt und einer Fettsäure oder Derivaten dieser; oder wobei
(3) das Arzneimittel ausgewählt wird als einem oder mehreren der folgenden Stoffe: einem kleinen Molekül, einem Protein und einem Peptid.

4. Verfahren nach einem der vorstehenden Ansprüche, wobei der Ballaststoff ausgewählt wird aus einem oder mehreren der folgenden Stoffe: einem löslichen Ballaststoff, einem präbiotischen Ballaststoff, Polydextrose und löslicher Maisfaser.

5. Verfahren nach einem der vorstehenden Ansprüche, wobei der matrixbildende Inhaltsstoff ferner Molkeproteinisolat und/oder Molkeproteinkonzentrat umfasst.

6. Verfahren nach einem der vorstehenden Ansprüche, wobei der eine oder die mehreren matrixbildenden Inhaltsstoffe im Bereich von 30 bis 100 Gewichtsprozent der Inhaltsstoffe insgesamt vorhanden sind, wenn die eine oder die mehreren Flüssigkeiten ausgenommen werden; vorzugsweise liegt der Anteil im Bereich von 40 bis 100 Gewichtsprozent der Inhaltsstoffe insgesamt.

7. Verfahren nach einem der vorstehenden Ansprüche, wobei (a) die Flüssigkeit ausgewählt wird aus Wasser, einer wässerigen Lösung, einem Alkohol oder einem verflüssigten Gas, optional Sauerstoff oder Stickstoff; und (b) ein Gas in fester Form ferner mit der einen oder den mehreren Flüssigkeiten den Feststoff-Inhaltsstoffen hinzugefügt wird, wobei es sich bei dem Gas in fester Form vorzugsweise um Kohlendioxid handelt.

8. Verfahren nach einem der vorstehenden Ansprüche, wobei die Inhaltsstoffe ferner folgendes umfassen:
einen Modifikator der Matrix, der ausgewählt wird aus der Gruppe, die einen oder mehrere der folgenden Stoffe umfasst: ein Milchpulver, einen Zucker, ein Mehl, eine Kleie, eine Stärke, einen Samen, einen gemahlenen Samen, eine gemahlene Hülsenfrucht, eine gemahlene Bohne, eine gemahlene Erbse, ein Fett und ein Öl.

9. Verfahren nach einem der vorstehenden Ansprüche, wobei die Inhaltsstoffe ferner einen oder mehrere der folgenden Stoffe umfassen: einen Geschmacksstoff, einen Farbstoff und ein Konservierungsmittel; wobei der Geschmacksstoff optional ausgewählt wird aus einem oder mehreren der folgenden Stoffe: einem künstlichen Geschmacksstoff, einem Pflanzenextrakt, einem getrockneten Pflanzenpulver, einem Salz, einem Süßstoff und einem Zucker.

10. Verfahren nach einem der vorstehenden Ansprüche, wobei die Behandlung der Stücke das Erwärmen oder Abkühlen über einen Zeitraum umfasst; wobei das Erwärmen es optional umfasst, dass die Teile einer oder mehreren der folgenden Optionen ausgesetzt werden:
Mikrowellenerwärmung, Konvektionserwärmung, leitender Erwärmung, Infraroterwärmung und dielektrischer Erwärmung.

11. Verfahren nach einem der vorstehenden Ansprüche, wobei es die Behandlung der Stücke ermöglicht, dass die Stücke über einen Zeitraum auf einer gewünschten Temperatur ruhen können, der ausreicht, damit die Stücke die gewünschte Temperatur erreichen können.

12. Verfahren nach einem der vorstehenden Ansprüche, wobei Schritt (a) auf Umgebungstemperatur ausgeführt wird, wie z.B. auf einer Temperatur im Bereich von 10 °C bis 25 °C.

13. Verfahren nach einem der vorstehenden Ansprüche, wobei das Aussetzen der Stücke zumindest des teilweisen Vakuums auf einer Temperatur im Bereich von 15 °C bis 75 °C erfolgt; vorzugsweise im Bereich von 15 °C bis 60 °C; wobei ein Bereich von 15 °C bis 55 °C darüber hinaus bevorzugt wird; wobei eine Temperatur im Bereich von 35 °C bis 55 °C noch mehr bevorzugt wird.

14. Verfahren nach einem der vorstehenden Ansprüche, wobei vor dem Formen in Stücke die vereinten Inhaltsstoffe gemeinsam über einen Zeitraum ruhen können, der ausreicht, um ein Gleichgewicht der Flüssigkeit und der sonstigen Inhaltsstoffe zu erzielen; wobei das Ruhen der Mischung der Inhaltsstoffe in einem geschlossenen Behälter oder in einer Umgebung mit Feuchtigkeitsregelung erfolgt.

15. Verfahren nach einem der vorstehenden Ansprüche, wobei:
(1) das erweiterte Nahrungsmittel zumindest unter Teilvakuumbedingungen und auf einer Temperatur getrocknet wird, die 75 °C nicht überschreitet; vorzugsweise auf einer Temperatur, die 60 °C nicht überschreitet; wobei eine Temperatur bevorzugt wird, die 55 °C oder weniger nicht überschreitet; und/oder
(2) das Verfahren ferner den Schritt des Kühlens des erweiterten Nahrungsmittels umfasst; wobei das Kühlen vorzugsweise zumindest unter Teilvakuumbedingungen erfolgt; und/oder
(3) das mindestens teilweise Vakuum im Bereich von 0,13 kPA bis 13,4 kPA liegt, vorzugsweise zwischen 0,67 kPa bis 8 kPa.

## Revendications

1. Procédé de production d'un produit alimentaire expansé ayant une structure interne de poches d'air, comprenant les étapes consistant à :
(a) combiner ensemble des ingrédients comprenant un ingrédient de formation de matrice choisi parmi les fibres alimentaires et/ou la farine de blé hydrolysée ;
(b) ajouter un ou plusieurs liquides aux ingrédients pour produire une pâte ou poudre humide à une température ne dépassant pas 75 °C ;
(c) former la pâte ou poudre humide en morceaux individuels ;
(d) traiter les morceaux afin qu'ils atteignent une température ne dépassant pas 75 °C, température à laquelle l'évaporation du liquide et/ou la transition de l'état solide à l'état gazeux est suffisante pour expanser les morceaux lorsqu'ils sont soumis à au moins un vide partiel ; et
(e) exposer les morceaux à au moins un vide partiel et à une température ne dépassant pas 75 °C de telle sorte que le liquide s'évapore amenant la matrice à s'expanser pour former le produit alimentaire ayant une structure interne de poches d'air.

2. Procédé selon la revendication 1, dans lequel les ingrédients comprennent en outre un ingrédient non structurel ; de préférence un ingrédient non structurel sensible à la température ; de manière davantage préférée :
(1) dans lequel l'ingrédient non structurel comprend un ou plusieurs d'un complément alimentaire, d'un nutraceutique, d'un probiotique, d'un produit pharmaceutique, d'un arôme, d'un colorant et d'un conservateur ; et/ou
(2) dans lequel l'ingrédient sensible à la température a une température seuil à une température dans la plage de 10 °C à 75 °C, en dessous de ladite température seuil, il reste sensiblement inchangé eu égard à un ou plusieurs de ses structure chimique, arôme, activité biologique, et activité pharmacologique.

3. Procédé selon la revendication 1 ou 2, dans lequel :
(1) le complément alimentaire est choisi parmi un ou plusieurs d'une vitamine, d'un minéral, d'une fibre, d'un extrait de plante, d'un acide gras et d'un acide aminé, ou de leurs dérivés ;
(2) le nutraceutique est choisi parmi un ou plusieurs d'un antioxydant, d'une fibre alimentaire soluble, d'un extrait de plante, et d'un acide gras, ou de leurs dérivés ; ou
(3) le produit pharmaceutique est choisi parmi un ou plusieurs d'une petite molécule, d'une protéine et d'un peptide.

4. Procédé selon l'une quelconque des revendications précédentes, dans lequel la fibre alimentaire est choisie parmi un ou plusieurs d'une fibre alimentaire soluble, d'une fibre alimentaire prébiotique, de polydextrose et d'une fibre de maïs soluble.

5. Procédé selon l'une quelconque des revendications précédentes, dans lequel l'ingrédient de formation de matrice comprend en outre un isolat de protéines de lactosérum et/ou un concentré de protéines de lactosérum.

6. Procédé selon l'une quelconque des revendications précédentes, dans lequel le ou les plusieurs ingrédients de formation de matrice sont présents dans la plage de 30 % à 100 % en poids du total des ingrédients à l'exclusion du ou des plusieurs liquides ; de préférence de 40 % à 100 % en poids dudit total des ingrédients.

7. Procédé selon l'une quelconque des revendications précédentes, dans lequel (a) le liquide est choisi parmi l'eau, une solution aqueuse, un alcool ou un gaz liquéfié, éventuellement l'oxygène ou l'azote ; et (b) un gaz à l'état solide est également ajouté avec le ou les plusieurs liquides aux ingrédients solides, de préférence le gaz solide est du dioxyde de carbone.

8. Procédé selon l'une quelconque des revendications précédentes, dans lequel les ingrédients comprennent en outre un agent de modification de la matrice choisi dans le groupe constitué par un ou plusieurs d'une poudre de lait, d'un sucre, de la farine, du son, d'un amidon, d'une graine, d'une semence, d'un légume sec moulu, d'une fève moulue, d'un pois moulu, d'une graisse et d'une huile.

9. Procédé selon l'une quelconque des revendications précédentes, dans lequel les ingrédients comprennent en outre un ou plusieurs d'un arôme, d'un colorant et d'un conservateur ; éventuellement, dans lequel l'arôme est choisi parmi un ou plusieurs d'un arôme artificiel, d'un extrait de plante, d'une poudre de plante sèche, d'un sel, d'un édulcorant et d'un sucre.

10. Procédé selon l'une quelconque des revendications précédentes, dans lequel le traitement des morceaux comprend le réchauffage ou le refroidissement pendant une période de temps ; éventuellement dans lequel le chauffage comprend l'exposition des morceaux à un ou plusieurs d'un chauffage à micro-ondes, chauffage par convection, chauffage par conduction, chauffage infrarouge et chauffage diélectrique.

11. Procédé selon l'une quelconque des revendications précédentes, dans lequel le traitement des morceaux leur permet de reposer pendant une période de temps à la température souhaitée suffisante pour que la température voulue soit atteinte par les morceaux.

12. Procédé selon l'une quelconque des revendications précédentes, dans lequel l'étape (a) est effectuée à une température ambiante, par exemple, une température dans la plage de 10 °C à 25 °C.

13. Procédé selon l'une quelconque des revendications précédentes, dans lequel l'exposition des morceaux à au moins un vide partiel est à une température dans la plage de 15 °C à 75 °C, de préférence dans la plage de 15 °C à 60 °C, de manière davantage préférée dans la plage de 15 °C à 55°C, et de manière encore davantage préférée une température dans la plage de 35 °C à 55 °C.

14. Procédé selon l'une quelconque des revendications précédentes, dans lequel avant la formation en morceaux, les ingrédients combinés sont laissés à reposer ensemble pendant une période de temps suffisante pour qu'un équilibrage du liquide et des autres ingrédients ait lieu ; de préférence dans lequel le repos du mélange des ingrédients a lieu dans un récipient scellé ou dans un environnement à humidité contrôlée.

15. Procédé selon l'une quelconque des revendications précédentes, dans lequel :
(1) le produit alimentaire expansé est séché sous au moins un vide partiel et à une température ne dépassant pas 75 °C, de préférence à une température ne dépassant pas 60 °C, de manière davantage préférée à une température ne dépassant pas 55 °C ou moins ; et/ou
(2) comprenant en outre l'étape consistant à refroidir le produit alimentaire expansé, de préférence dans lequel le refroidissement est sous au moins un vide partiel ; et/ou
(3) l'au moins vide partiel est dans la plage de 0,13 kPa à 13,4 kPa, de préférence de 0,67 kPa à 8 kPa.
